# EUROPEAN PATENT APPLICATION

(11) **EP 4 467 979 A1**
(43) Date of publication of application: **27.11.2024**
(21) Application number: 23746998.6
(22) Date of filing: 25.01.2023
(51) Int. Cl.: G01N 33/53, G01N 33/543, G01N 33/569

(54) **METHOD FOR DETECTING HELICOBACTER SUIS ANTIBODY USING BACTERIAL OUTER MEMBRANE FRACTION**

(30) Priority: 25.01.2022 JP 2022009302
(71) Applicant: The Kitasato Institute, Tokyo 108-8641 (JP); Japan as Represented by Director-General of National Institute of Infectious Diseases, Tokyo 162-8640 (JP); Denka Company Limited, Tokyo 103-8338 (JP)
(72) Inventor: MATSUI, Hidenori, Tokyo 108-8641 (JP); RIMBARA, Emiko, Musashimurayama-shi, Tokyo 208-0011 (JP); SUZUKI, Masato, Higashimurayama-shi, Tokyo 189-0002 (JP); TAKAHASHI, Takamichi, Gosen-shi, Niigata 959-1695 (JP); ISHII, Akira, Gosen-shi, Niigata 959-1695 (JP)
(74) Representative: Hoffmann Eitle
(86) International application number: PCT/JP2023/002329
(87) International publication number: WO 2023/145788

(57) **Abstract**

Provided are a method and a reagent for measuring an antibody against *Helicobacter suis* using an outer membrane fraction of *H. suis.* A reagent for detecting an antibody that binds to *a H. suis* outer membrane fraction, or a protein contained in the outer membrane fraction, of a specimen derived from a subject, the reagent comprising at least the *H. suis* outer membrane fraction, or the protein contained in the outer membrane fraction, and an anti-Ig antibody.

## Description

### Technical Field

The present invention relates to a method for measuring an antibody against *Helicobacter suis* using *a H. suis* outer membrane fraction.

### Background Art

It is now known that infection with *Helicobacter pylori* (a microaerophilic gram-negative spiral bacterium that parasitizes the human stomach, hereafter referred to as "*H. pylori*") is involved in chronic gastritis, gastric ulcers, duodenal ulcers, gastric cancer, gastric mucosa-associated lymphoid tissue (MALT) lymphoma, diffuse large B-cell lymphoma, and the like. The main methods used to test for *H. pylori* infection are an isolation and culture method, urea breath test (UBT), measurement of *H. pylori* antibody titers in serum and urine (ELISA and latex agglutination method), measurement of *H. pylori* antigens in the stool (immunochromatography), and rapid urease test (RUT) of gastric biopsy specimens.

However, in recent years, as the infection rate of *H. pylori* has decreased due to the widespread diagnosis and eradication of *H. pylori,* non*-Helicobacter pylori* hericobacters (NHPH, Helicobacters other than *H. pylori*) have become a problem as a type of Helicobacter that induces severe gastric diseases in humans other than *H. pylori.* NHPH includes *Helicobacter suis* (from now on referred to as *H. suis*), *H. bizzozeronnii, H. felis, H. salmonis, H. ailurogastricus, H. cynogasticus, H. baculiformis, H. mustelae, H. acinonychls, H. cetorum,* and *H. heilmannii,* and among them, the majority found in the human stomach is *H. suis.*

*H. pylori* only infects primates, and infection from close relatives is assumed to occur only during infancy, whereas *H. suis* can be transmitted from animals such as pigs and monkeys regardless of age.

*H. suis* was a parasite that lived in monkeys' stomachs about 100,000 years ago. Then, pigs began to be infected 15,000 years ago. As pigs were domesticated, the infection spread explosively, and humans also began to be infected (Non-Patent Literature 1). Multi-Locus Sequencing Typing (MLST) analysis of the *H. suis* genes of 181 strains isolated from around the world showed that independent clusters were formed depending on the infected host animal, but the strains isolated from humans did not form independent clusters and were included in the pig cluster, and thus it was determined that the source of infection for humans was pigs (Non Patent Literature 2). Therefore, diagnosis and eradication of the infection are necessary for pigs and humans.

As a method for testing for *H. suis* infection, the measurement of *H. suis* antibody titers in serum (ELISA and latex agglutination method) has been reported (Patent Literatures 1 and 2).

### Citation List

### Patent Literature

Patent Literature 1: JP Patent Publication (Kokai) 2016-10331 A
Patent Literature 2: International Publication No. WO 2019/225639

### Non-Patent Literature

Non-Patent Literature 1: Flahou et al., ISM J., Jan;12(1):77-86. doi:10.1038/ismej. 2017.145
Non-Patent Literature 2: E. Rimbara et al., Proc Natl Acad Sci USA 2021 Vol. 118 Issue 13 e2026337118. doi: 10.1073/pnas. 2026337118.
Non-Patent Literature 3: Haesebrouck F. et al., Helicobacter, 16(4), 339-340, 2011, doi:10.1111/j. 1523-5378.2011.00849. x
Non-Patent Literature 4: A. D. Augustin, et al., Front. Med., 2019, 6, 188, doi: 10.3389/fmed. 2019.00188 (https://www.ncbi.nlm.nih.gov/pubmed/31555648)

### Summary of Invention

### Technical Problem

An object of the present invention is to provide a method and a reagent for measuring an antibody against *Helicobacter suis* using an outer membrane fraction of *H. suis.*

### Solution to Problem

Patent Literature 1 discloses immobilizing the F2R2 protein of *H. suis* on a plate to measure an antibody in serum. Patent Literature 2 discloses immobilizing the HsvA protein, which is said to have a higher antibody titer than the F2R2 protein, on a plate to measure an antibody in serum.

However, there has been a demand for a method with higher sensitivity and specificity than conventional methods.

The present inventors have found a novel measurement method and have found that the use of *a H. suis* outer membrane fraction or a protein contained in the outer membrane fraction can improve the sensitivity and specificity of measuring an anti-H. *suis* antibody in the bodies of infected individuals.

More specifically, the present invention relates to the following inventions.
[1] A reagent for detecting an antibody that binds to *a H. suis* outer membrane fraction, or a protein contained in the outer membrane fraction, in a specimen derived from a subject, the reagent comprising at least the *H. suis* outer membrane fraction, or the protein contained in the outer membrane fraction, and an anti-Ig antibody.
[2] According to [1], the reagent wherein the subject is a mammal.
[3] The reagent, according to [1] or [2], wherein the specimen is derived from blood.
[4] The reagent, according to any one of [1] to [3], for detecting also an antibody that binds to *H. pylori,* or an antigen of an *H. pylori* cell component, in the specimen derived from a subject, the reagent further comprising an *H. pylori* cell component or an antibody against the *H. pylori* cell component.
[5] The reagent according to any one of [1] to [4], wherein the reagent is a reagent for ELISA or immunochromatography.
[6] A method for detecting an antibody that binds to an anti-*H*. *suis* outer membrane fraction, or a protein contained in the outer membrane fraction, in a specimen derived from a subject, the method comprising the steps of:
   (a) contacting a specimen derived from a subject with *a H. suis* outer membrane fraction or a protein contained in the outer membrane fraction and
   (b) detecting an antibody bound to the *H. suis* outer membrane fraction, or the protein contained in the outer membrane fraction, in the specimen.
[7] According to [6], the method is wherein the subject is a mammal.
[8] The method, according to [6] or [7], wherein the specimen is derived from blood.
[9] The method, according to any one of [6] to [8], for detecting also an antibody that binds to *H. pylori* in the specimen derived from a subject, the method further comprises the steps :
   (a) contacting a specimen derived from a subject with an *H. pylori* cell component and
   (b) detecting an antibody bound to the *H. pylori* cell component in the specimen.
[10] The method, according to any one of [6] to [8], for detecting also an antigen of an *H. pylori* cell component in the specimen derived from a subject, the method further comprises the steps:
   (a) contacting a specimen derived from a subject with an antibody against an *H. pylori* cell component and
   (b) detecting an antigen of an *H. pylori* cell component bound to the antibody against the *H. pylori* cell component in the specimen.
[11] A method for detecting infection with *H. suis,* wherein a subject is determined to be infected with *H. suis,* in which an antibody is detected by measurement using the reagent according to any one of [1] to [5] and the method according to any one of [6] to [10].

The present description includes the disclosure of JP Patent application No. 2022-009302, which is the priority basis of the present application.

### Advantageous Effects of Invention

Using *a H. suis* outer membrane fraction, or a protein contained in the outer membrane fraction, can measure an anti-*H*. *suis* antibody in the body of infected individuals with high sensitivity. The *H. suis* outer membrane fraction, or the protein contained in the outer membrane fraction, can be used to measure the presence or absence or antibody titer of the anti-*H*. *suis* antibody in blood derived from a subject diagnosed with *H. suis* infection.

### Brief Description of Drawings

[Figure 1] Figure 1 is a view showing the measured values of the ELISA results using human serum (diluted 3600-fold) as a specimen and *H. suis* whole cells or *a H. suis* outer membrane fraction as an antigen.
[Figure 2] Figure 2 is a graph showing the results of ELISA using human serum (diluted 3600-fold) as a specimen and *H. suis* whole cells (Figure 2A) or *a H. suis* outer membrane fraction (Figure 2B) as an antigen.
[Figure 3] Figure 3 is a view showing the measured values of the results of ELISA using mouse serum (diluted 3600-fold) as a specimen and *H. suis* whole cells or *a H. suis* outer membrane fraction as an antigen.
[Figure 4] Figure 4 is a graph showing the results of ELISA using mouse serum (diluted 3600-fold) as a specimen and *H. suis* whole cells (Figure 4A) or *a H. suis* outer membrane fraction (Figure 4B) as an antigen.
[Figure 5] Figure 5 is a view showing the measured values of the results of ELISA using human serum (diluted 3600-fold) as a specimen and an *H. pylori* outer membrane fraction or *a H. suis* outer membrane fraction as an antigen.
[Figure 6] Figure 6 is a graph showing the results of ELISA using human serum (diluted 3600-fold) as a specimen and an *H. pylori* outer membrane fraction (Figure 6A) or *a H. suis* outer membrane fraction (Figure 6B) as an antigen.
[Figure 7-1] Figure 7-1 is a view showing the measured values of the results of ELISA using human serum (diluted 3600-fold) as a specimen and *a H. suis* outer membrane fraction or a partial peptide of HsvAof *H. suis* (SEQ ID NOs: 1 to 5) as antigens.
[Figure 7-2] Figure 7-2 is a view showing the measured values of the ELISA results using human serum (diluted 3600-fold) as a specimen and *a H. suis* outer membrane fraction or partial peptides of HsvA of *H. suis* (SEQ ID NOs: 6 to 11) as an antigen.

### Description of Embodiments

From now on, the method of the present invention will be described.

The present invention is a method for detecting an antibody against *a H. suis* cell outer membrane fraction or a protein contained in the outer membrane fraction in a biological sample from a subject, and a reagent for detecting the antibody. The present invention is also a method for determining the presence of *H. suis* in a subject, or a method for detecting *H. suis* infection in a subject. Alternatively, the present invention is a method for obtaining auxiliary data for diagnosing *a H. suis* infection in a subject.

### (Helicobacter suis: H. suis)

As used herein, *"H. suis"* refers to the *H. suis* species included in non-*Helicobacter pylori* helicobacters (NHPH, Helicobacters other than *H. pylori*) or *Helicobacter heilmannii sensu lato* (*Helicobacter heilmannii* in the broad sense) among the more than 50 reported species of Helicobacter bacteria (Non-Patent Literature 3). *H. suis* is known to infect the stomachs of pigs, monkeys, wild boars, cats, dogs, and other animals in addition to humans. The infected mammal from which *H. suis* is isolated in the present description is not limited and may be, for example, a human, monkey, wild boar, or pig.

### (H. suis outer membrane fraction or protein contained in outer membrane fraction)

As used herein, the term *"H. suis* outer membrane fraction" is prepared by collecting *H. suis* cell outer membrane fragments after ultrasonic disruption of *H. suis.* Ultrasonic disruption can be performed by suspending *H. suis* cells in a buffer solution and using an ultrasonic disrupter. Examples of the ultrasonic disrupter include a sample-sealed ultrasonic disruption device, Bioruptor (BM Equipment Co., Ltd.). Ultrasonic disruption destroys *H. suis* cells, leaving behind outer membrane fragments. The outer membrane fragment of the cell can be collected by centrifugation or filtration. "Protein contained in *a H. suis* outer membrane fraction" refers to a protein derived from *H. suis* present in a fraction prepared by collecting *H. suis* cell outer membrane fragments after ultrasonic disruption of *H. suis.*

### (Method and reagent for detecting antibody against H. suis outer membrane fraction or protein contained in outer membrane fraction)

The antibody against the *H. suis* outer membrane fraction refers to an antibody against an antigen, such as a protein or sugar contained in the *H. suis* outer membrane fraction, and may include a plurality of antibodies against various substances. In the present invention, an antibody against at least one of antigens such as a protein and sugar contained in the *H. suis* outer membrane fraction is called an antibody against the *H. suis* outer membrane fraction. In addition, the antibody against a protein in *a H. suis* outer membrane fraction refers to an antibody against a protein among the antigens contained in the *H. suis* outer membrane fraction. The *H. suis* outer membrane fraction contains multiple proteins, and the antibody against a protein contained in *a H. suis* outer membrane fraction may include multiple antibodies against various proteins. In the present invention, an antibody against at least any of the proteins contained in the *H. suis* outer membrane fraction is referred to as an antibody against a protein contained in *a H. suis* outer membrane fraction. In actuality, in a subject infected with *H. suis,* the breakdown of *H. suis* bacteria in the body results in the production of the antibody against the *H. suis* outer membrane fraction, or the antibody against a protein contained in the outer membrane fraction in the subject.

The binding of an antibody to an antigen contained in *a H. suis* outer membrane fraction is referred to as the binding of the antibody to the *H. suis* outer membrane fraction.

A reagent for detecting an antibody against the *H. suis* outer membrane fraction, or a protein contained in the outer membrane fraction, can be prepared as a reagent applicable to known methods. Examples of known methods include: a labeled immunoassay method such as an enzyme immunoassay method (EIA method), a simplified EIA method, an enzyme-linked immunosorbent assay method (ELISA method), a radioimmunoassay method (RIA method), and a fluorescent immunoassay method (FIA method); an immunoblotting method; an immunochromatography method; a chromatography method; a turbidimetry method (TIA method); a nephelometry method (NIA method); a colorimetry method; a latex agglutination method (LIA method); a particle counting method (CIA method); a chemiluminescence assay method (CLIA method, CLEIA method); a precipitation reaction method; a surface plasmon resonance method (SPR method); a resonant mirror detector method (RMD method); and a comparative interference method. Whether or not the reagent of the present invention can be applied to a desired measurement method can be confirmed by measuring whether or not the detection is possible by performing each measurement method using a specimen containing an antibody against *a H. suis* outer membrane fraction, or a protein contained in the outer membrane fraction, or a sample containing an antibody against *a H. suis* outer membrane fraction, or a protein contained in the outer membrane fraction, with the same concentration as the specimen.

The antibody against *a H. suis* outer membrane fraction, or a protein contained in the outer membrane fraction, can be used as a marker for *H. suis* infection.

That is, whether or not a subject is infected with *H. suis* can be determined by confirming the presence of an antibody against the *H. suis* outer membrane fraction or a protein contained in the outer membrane fraction produced by the subject's immune system. The presence of this antibody can be detected and confirmed by antigen-antibody reactions using *a H. suis* outer membrane fraction, or a protein contained in the outer membrane fraction. Detection of an antibody against *a H. suis* outer membrane fraction, or *a H. suis* outer membrane fraction using the *H. suis* outer membrane fraction, or the protein contained in the outer membrane fraction, as a measurement reagent can be performed by an immunological assay method such as a labeled immunoassay method including an enzyme immunoassay method (EIA method), a simplified EIA method, an enzyme-linked immunosorbent assay method (ELISA method), a radioimmunoassay method (RIA method), and a fluorescent immunoassay method (FIA method); an immunoblotting method including a Western blotting method; an immunochromatography method including a colloidal gold agglutination method; a chromatography method including an ion exchange chromatography method and affinity chromatography method; a turbidimetry method (TIA method); a nephelometry method (NIA method); a colorimetry method; a latex agglutination method (LIA method); a particle counting method (CIA method); a chemiluminescence method (CLIA method, CLEIA method); a sedimentation reaction method; a surface plasmon resonance method (SPR method); a resonant mirror detector method (RMD method); and a comparative interference method.

In one aspect, the present invention relates to a method for determining *H. suis* infection, comprising the following steps:
(a) contacting a specimen derived from a subject with *a H. suis* outer membrane fraction or a protein contained in the outer membrane fraction;
(b) detecting an antibody bound to the *H. suis* outer membrane fraction, or the protein contained in the outer membrane fraction, in the specimen; and
(c) determining a subject to be infected with *H. suis,* in which the antibody bound to the *H. suis* outer membrane fraction, or the protein contained in the outer membrane fraction is detected, and determining a subject to be not infected with *H. suis,* in which the antibody bound to the *H. suis* outer membrane fraction, or the protein contained in the outer membrane fraction is not detected.

In another aspect, the present invention relates to a method for determining *H. suis* infection, comprising the following steps:
(a) contacting a specimen derived from a subject with *a H. suis* outer membrane fraction, or a protein contained in the outer membrane fraction;
(b) measuring a level of an antibody bound to the *H. suis* outer membrane fraction, or the protein contained in the outer membrane fraction, in the specimen, and
(c) determining a subject to be infected with *H. suis,* who has the measured level of the antibody that is higher than a level of the antibody measured in a negative control by the same method, and determining a subject to be not infected with *H. suis,* who has the measured level of the antibody that is equal to or lower than a level of the antibody measured in a negative control by the same method. Herein, the negative subject refers to a specimen derived from a subject who is not infected with *H. suis.* The antibody level refers to the antibody's quantitative value, i.e., the antibody titer, and is represented, for example, as the concentration in the specimen. In addition, the measurement of the level of the antibody is included in the detection of the antibody.

Whether or not *a H. suis* outer membrane fraction, or a protein contained in the outer membrane fraction is bound to an antibody in a specimen derived from a subject may be confirmed by the following illustrative method. For example, *a H. suis* outer membrane fraction, or a protein contained in the outer membrane fraction, is bound to a carrier for immobilization, and a specimen derived from a subject is contacted with the *H. suis* outer membrane fraction, or the protein contained in the outer membrane fraction. Then, the reaction system is washed to remove unbound antibodies. As a result, the reaction system includes an antibody complex in the specimen derived from the subject, bound to the *H. suis* outer membrane fraction of the protein contained in the outer membrane fraction. Then, after contacting with a labeled anti-Ig antibody to allow the antibody derived from the subject, bound to the *H. suis* outer membrane fraction, or the protein contained in the outer membrane fraction, to bind the labeled anti-Ig antibody, the reaction system is washed to remove the unbound labeled anti-Ig antibody, and the label of the remaining labeled anti-Ig antibody is detected. Suppose the anti-Ig antibody label is detected. In that case, it can be determined that the antibody in the specimen derived from the subject is bound to the *H. suis* outer membrane fraction or the protein contained in the outer membrane fraction. Measuring the level of the labeled anti-Ig antibody can also measure the level of binding of the *H. suis* outer membrane fraction, or the protein contained in the outer membrane fraction, to the antibody in the specimen derived from the subject. The anti-Ig antibody may be an anti-IgG antibody or an anti-IgM antibody and is preferably an anti-IgG antibody. In addition, a monoclonal antibody is preferably used as the anti-IgG antibody. In addition, fragments having specific antigen-binding ability (antigen-binding pieces), such as Fab, Fab', F(ab')₂, single-chain antibody (scFv), VHH single domain antibody (nanobody), dsFv, diabody, and minibody, can also be used. Examples of such methods include ELISA and the like and an immunochromatography method. For the label of the anti-Ig antibody, there are often used enzymes such as alkaline phosphatase and horseradish peroxidase, metal colloids such as gold colloids, silica particles, cellulose particles, magnetic particles, fluorescent particles, colored polystyrene particles, and colored latex particles. When colored particles such as metal colloid particles, colored polystyrene particles, or colored latex particles are used, coloring occurs due to aggregation of these labeling reagents, and thus this coloring is measured. In a case where the level of the binding is used as an index, the amount of antibody can be calculated from the measured value by creating a standard curve with a standard solution having the already known amount. Alternatively, a surface plasmon resonance sensor can be used to detect or measure the binding of the *H. suis* outer membrane fraction, or the protein contained in the outer membrane fraction, to an antibody in a specimen derived from a subject.

Among the above immunoassay methods, the sandwich method is preferable. The sandwich method itself is well known in the field of immunoassay and can be performed, for example, by an immunochromatography method or ELISA method, which performs immunoassay in a lateral flow format. All of these sandwich methods are well known, and the method of the present invention can be performed by the well-known sandwich methods. The ELISA method will be explained below.

The *H. suis* outer membrane fraction, or the protein (antigen) contained in the outer membrane fraction, is immobilized on an ELISA plate, and a specimen that may contain an antibody against the *H. suis* outer membrane fraction, or the protein contained in the outer membrane fraction, is added and contacted with the immobilized carrier to form an antigen-antibody complex on the immobilized carrier. Further, an enzyme-labeled antibody specific to the human antibody (anti-Ig antibody) is added and contacted to cause the anti-Ig antibody to bind to the antigen-antibody complex on the immobilized carrier. Then, a substrate for the enzyme is added to perform the enzyme reaction, and the color generated is measured by measuring absorbance to detect the sandwich complex of the antigen and antibody on the plate.

The antigen-antibody reaction can be performed at 4°C to 45°C, preferably 20°C to 40°C, and more preferably 25°C to 38°C. The reaction time for each binding reaction is about 10 minutes to 18 hours, more preferably 10 minutes to 3 hours, and still more preferably about 30 minutes to 2 hours.

The binding of the *H. suis* outer membrane fraction or the protein (antigen) contained in the outer membrane fraction to a carrier can be performed by known methods such as physical adsorption or covalent bonds using functional groups. The amount of immobilization is relatively limited, and if the carrier is a 96-well microtiter plate, a few ng to several tens of µg per well is desirable. Immobilization can be performed by contacting a solution of the *H. suis* outer membrane fraction, or the protein (antigen) contained in the outer membrane fraction, to be immobilized with the carrier. For example, a solution of the *H. suis* outer membrane fraction, or the protein (antigen) contained in the outer membrane fraction can be dispensed into the wells of a microtiter plate and allowed to stand for a certain period of time to allow immobilization. After immobilizing the *H. suis* outer membrane fraction, or the protein (antigen) contained in the outer membrane fraction, blocking is performed using a blocking solution containing bovine serum albumin, human serum albumin, rabbit serum albumin, ovalbumin, or the like to prevent non-specific binding during the assay.

*H. suis* widely infects mammals, and thus the subject in the method for determining the presence of *H. suis* and the method for determining infection with *H. suis* of the present invention can be a mammal such as a human, monkey, pig, cat, wild boar, dog, rabbit, mouse, or sheep, and is preferably a human. There is more preferable the human patient suffering from gastritis, chronic gastritis, goose bump gastritis, type A gastritis, gastric MALT lymphoma, diffuse large B-cell lymphoma, gastric cancer, gastric/duodenal ulcer, idiopathic thrombocytopenic purpura, functional dyspepsia, or Parkinson's disease. In addition, the method of the present invention can be performed qualitatively, quantitatively, or semi-quantitatively (Non-Patent Literature 4).

As the specimen used in the method of the present invention for determining the presence of *H. suis,* for example, a tissue sample taken from a subject as a biopsy or a liquid sample taken from a subject can be used. The specimen is not particularly limited as long as it can be used as a target for the method of the present invention, and examples thereof include tissue, blood, plasma, serum, lymph, urine, feces, serous fluid, cerebrospinal fluid, synovial fluid, aqueous humor, tears, saliva, or fractions thereof or processed products thereof. In a case where an antibody is to be detected in the method of the present invention for determining the presence of *H. suis,* preferable specimens are blood, plasma, serum, lymph, and urine.

The present invention includes a kit for measuring antibodies against *a H. suis* outer membrane fraction, or a protein contained in the outer membrane fraction, in a specimen, the kit including at least a carrier on which the *H. suis* outer membrane fraction, or the protein contained in the outer membrane fraction, is immobilized, and further including a labeled anti-Ig antibody.

The *H. suis* outer membrane fraction, or the protein contained in the outer membrane fraction, of the present invention can be appropriately prepared by a method well known to those skilled in the art with reference to the disclosures of the present description. In addition, the reagent of the present invention can also be appropriately produced by a method well known to those skilled in the art.

In one aspect, the present invention relates to a method for obtaining a specimen and determining the presence of *H. suis* in a subject, comprising a method for detecting an antibody against *a H. suis* outer membrane fraction, or a protein contained in the outer membrane fraction, in the specimen. In particular, the present invention relates to a method for determining *H. suis* infection in a subject, comprising detecting an antibody against *a H. suis* outer membrane fraction, or a protein contained in the outer membrane fraction. In the present method, a subject from whom a specimen with the antibody detected against *a H. suis* outer membrane fraction, or a protein contained in the outer membrane fraction, is collected is determined to have *H. suis* or to be infected with *H. suis.*

In a case where the method of the present invention is performed by measuring the binding of the antibody in the specimen to the test reagent, whether "detected " or not does not necessarily mean absolute detection, but may be determined by comparison with other specimens. That is, the presence or absence of detection may be determined from the measured value, not based on the positive or negative detection result. That is, in the method of the present invention, the "detecting" step may be replaced with "measuring" as necessary, and whether "detected" or not may be determined based on the measured value of the target substance in comparison with a negative subject. For example, in a case where a target substance is detected in a negative subject, i.e., a specimen that does not contain *H. suis* or a specimen derived from a subject who is clearly not infected with *H. suis,* if the measured value of the test subject is equivalent to that of the negative subject, even with a small amount of the substance detected, it is determined that *H. suis* is not present or that the subject is not infected with *H. suis,* as "not detected" in the method of the present invention. On the other hand, if the measured value of the subject is higher than that of the negative subject, it is determined that *H. suis* is present or that the subject is infected with *H. suis,* as "detected". Therefore, in the method of the present invention, the observation of a small measured value for a negative subject is within the range preliminarily permitted by the present invention.

In the method of the present invention, the antibody titers of antibodies in specimens from *H. suis*-infected and *H. suis*-uninfected individuals can be measured and a cutoff value can be set. At the cutoff value or more, it is possible to determine that *H. suis* is present or that the subject is infected with *H. suis.*

The cutoff value can be determined, for example, by receiver operating characteristic curve (ROC) analysis. In addition, the diagnostic accuracy (sensitivity and specificity) of the method of the present invention can be determined by ROC analysis. In the ROC analysis, anti-H. *suis* antibodies are measured for specimens taken from *H. suis*-infected individuals and specimens taken from *H*. *suis*-uninfected individuals, and the sensitivity and false positive rate (1-specificity) at each cutoff value are calculated and plotted on a coordinate system with (1-specificity) on the horizontal axis and sensitivity on the vertical axis. In a case where the diagnostic accuracy is analyzed by ROC analysis, the sensitivity is 80% or more, preferably 85% or more, and more preferably 90% or more, and the specificity is 75% or more, preferably 80% or more.

### (Detection of antibody against H. pylori or H. pylori antigen)

The present invention also includes a method for detecting an antibody that binds to *a H. suis* outer membrane fraction, or a protein contained in the outer membrane fraction, and an antibody against *H. pylori* in the same subject, and a reagent used in the method. Herein, the antibody against *H. pylori* refers to any component of the *H. pylori* whole cell, i.e., an antibody against a cell component. For example, if the *H. pylori* test indicates negative but symptoms of stomach disease are present, the cause may be infection with *H. suis.* Detecting an antibody that binds to *a H. suis* outer membrane fraction, or a protein contained in the outer membrane fraction, and an antibody against *H. pylori* in the same specimen can confirm not only the presence or absence of *H. pylori* infection, but also *H. suis* infection in specimens that have previously been overlooked as *H. pylori* negative. Early confirmation of infection allows for the decision of appropriate treatment strategies. Specimens from the same subject may be the same or different from each other for *H. pylori* and *H. suis.* That is, detection of an antibody that binds to *a H. suis* outer membrane fraction, or a protein contained in the outer membrane fraction, and detection of an antibody against *H. pylori* may be performed simultaneously using the same specimen, or may be performed separately using different specimens collected at different times. Detection of an antibody against *H. pylori* may be performed in a manner similar to that used for detection of an antibody against *a H. suis* outer membrane fraction, or a protein contained in the outer membrane fraction.

In the determination of *H. pylori,* whether "detected" or not does not necessarily have to be an absolute detection, and may be determined by comparison with other specimens. That is, the presence or absence of detection may be determined from the measured value, not based on the positive or negative detection results. That is, in the method of the present invention, the step of "detecting" can be replaced with "measuring" as necessary, and whether "detected" or not may be determined based on the measured value of the target substance in comparison with a negative subject. For example, in a case where a target substance is detected in a negative subject, i.e., a specimen not containing *H. pylori* or a specimen derived from a subject who is not infected with *H. pylori,* if the measured value of the test subject is equivalent to the measured value of the negative subject, even with a small amount detected, it is determined that *H. pylori* is not present or that the subject is not infected with *H. pylori* as "not detected" in the method of the present invention. On the other hand, if the measured value of the subject is higher than that of the negative subject, it is determined that *H. pylori* is present or that the subject is infected with *H. pylori* as "detected". Therefore, in the method of the present invention, the observation of a small amount of measured value for a negative subject is within the range preliminarily permitted by the present invention.

The present invention includes a kit for measuring an antibody against *a H. suis* outer membrane fraction, or a protein contained in the outer membrane fraction, and an antibody against *H. pylori,* in a specimen, the kit comprising at least a carrier on which *a H. suis* outer membrane fraction, or a protein contained in the outer membrane fraction, is immobilized, and a carrier on which any of the components of the *H. pylori* whole cells is immobilized, and further comprising a labeled anti-human Ig antibody.

Further, an *H. pylori* antigen may be detected in a sample derived from a subject. Detection of the *H. pylori* antigen can also detect *H. pylori* infection. The sample for detecting the *H. pylori* antigen is preferably a feces. The present invention includes a kit for measuring an antibody against *a H. suis* outer membrane fraction, or a protein contained in the outer membrane fraction, and an *H. pylori* antigen in a specimen, the kit comprising at least a carrier on which *a H. suis* outer membrane fraction, or a protein contained in the outer membrane fraction, is immobilized, and a carrier on which an antibody against an *H. pylori* is immobilized, and further comprising a labeled anti-human Ig antibody.

### Examples

The present invention will be specifically explained below with reference to examples, but the present invention is not limited thereto. All references cited throughout the present application are incorporated herein by reference in their entirety.

### (Example 1) Measurement in human specimen

Measurement of anti-*H*. *suis* antibody titers in infected individuals (humans) using enzyme linked immunosorbent assay (ELISA)
Composition of H. Swiss medium
Brucella broth (BD BBL) 2.8 g
Agar powder (BD BBL) 1.5 g
Pyruvic acid (Sigma-Aldrich Co. LLC) 0.1 g, added for preparation of agar medium
Skirrow Supplement (2 mL/vial, Oxoid Ltd.) 0.4 mL
Vitox Supplement (10 mL/vial, Oxoid Ltd.) 2 mL
Amphotericin B (0.25 mg/mL) 2 mL
Concentrated hydrochloric acid 0.135 mL, for adjusting pH to 5
Inactivated fetal bovine serum (FBS) 20 mL
Distilled water was added to bring the total volume to 100 mL.

Nine mL of agar medium was added to a 75 cm² flask to prepare a slant medium, and *H. suis* SNTW101c strain (Non-Patent Literature 2) stored at -80°C and isolated from a patient with goosebump gastritis was inoculated thereinto, 12 mL of liquid medium was added, and shaking culture was performed for one week at a temperature of 37°C, 100% humidity, and under microaerobic conditions (5% O₂, 12% CO₂, 83% N₂). Thereafter, the culture medium was centrifuged (13,420 G × 10 minutes) to collect the cells. The cells were washed twice with phosphate buffered saline (PBS, pH 7.4), suspended in distilled water, and subjected to ultrasonic disruption treatment for 5 minutes under ice-cooled conditions (using a Bioruptor II ultrasonic cell disrupter (setting High), 30 seconds of sonication and 30 seconds of rest were repeated five times). The disrupted solution was regarded as an *"H. suis* whole cell suspension" and the sediment was centrifuged (30, 190 G × 10 minutes) to provide the *"H. suis* outer membrane fraction", which was then suspended in distilled water. Protein was quantified using the Bio-Rad Protein Assay kit with BSA as the standard protein.

A suspension of *H. suis* whole cells or *a H. suis* outer membrane fraction (4 µg/mL) dissolved in 0.1 M carbonate-bicarbonate buffer (pH 9.4) was dropped at 100 µL/well onto a 96-well NUNC ImmunoPlate #439454 and left at 4°C overnight. The next day, the cell solution was discarded and the wells were washed three times with 200 µL/well of PBS-T (PBS containing 0.05% (V/V) Tween 20). A blocking solution (1% (V/V) BSA, PBS-based, pH 7.4) prepared from Blocker BSA (bovine serum albumin) 10X in PBS (Thermo Fisher Scientific Inc.) was dropped at 200 µL/well and left at 37°C for 1 hour. The blocking solution was discarded, and washing was performed three times with 200 µL/well of PBS-T. Serum was collected from *H. pylori-*infected individuals (determined by existing testing methods (urea breath test, antibody test, antigen test, and the like)) and *H. suis*-infected individuals (DNA was prepared from a gastric biopsy specimen of the subject, and *H. suis* infection was determined by the real-time PCR method described in Patent Literature 2 or by culturing the gastric biopsy specimen). As a control, serum was collected from a healthy individual not infected with any of the cells (uninfected). Human serum diluted with blocking solution was dropped at 50 µL/well and left at 37°C for 1 hour. The serum solution was discarded and washing was performed three times with 200 µL/well of PBS-T. Horseradish peroxidase labeled secondary antibody (Goat anti-Human IgA+IgG+IgM (H+L), Jackson ImmunoResearch, Inc.) diluted 100,000-fold using blocking solution was dropped at 50 µL/well and left at 37°C for 1 hour. The secondary antibody solution was discarded, and washing was performed three times with 200 µL/well of PBS-T. SuperBlue TMB Microwell Peroxidase Substrate (1-Component), Kirkegaard & Perry Laboratories, Inc. (KPL) was dropped at 50 µL/well to produce a blue color, and then 1 N hydrochloric acid was dropped at 50 µL/well to cause the color to turn yellow. The absorbance at 450 nm (reference wavelength: 620 nm to 630 nm) was measured using a plate reader.

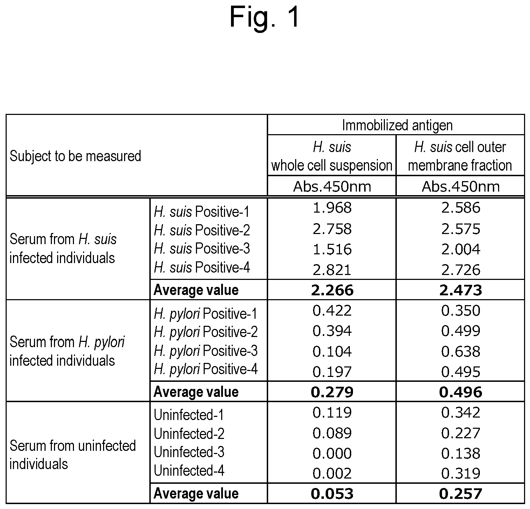

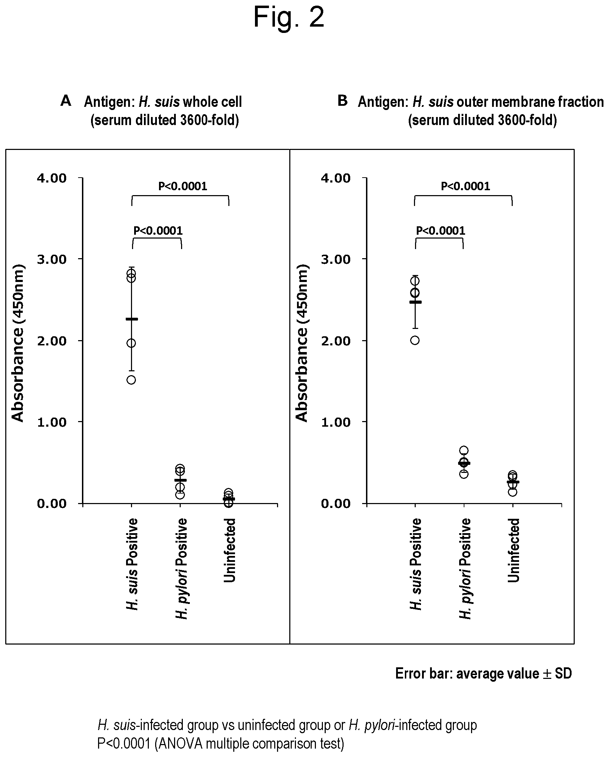

The results of ELISA using human serum (diluted 3600-fold) are shown in Figures 1 and 2. *H. suis*-positive subjects showed absorbance for both the *H. suis* whole cell antigens and the *H. suis* solubilized fraction, but the *H. suis* solubilized fraction showed a higher absorbance. In addition, *H. pylori-*infected individuals and uninfected individuals who were not infected with *H. suis* had low absorbance for both antigens. This demonstrated for the first time that an antibody against the *H. suis* solubilized fraction is produced in the blood of *H. suis*-infected individuals, and that the use of the *H. suis* solubilized fraction as an antigen allows for highly sensitive capture of the antibody. This demonstrated that using the *H. suis* solubilized fraction allows for distinction from *H. pylori* infection and thus a highly sensitive diagnosis of *H. suis* infection in humans.

### (Example 2) Measurement in mammalian specimens

The cultured *H. suis* SNTW101c strain was centrifuged to collect the cells. The cells were suspended in PBS and orally administered once to 4-week-old female C57BL/6 mice at 1 × 10⁸ colony forming units (CFU). On the other hand, *H. pylori* SS1 strain stored at -80°C was inoculated onto Nissui plate Helicobacter agar medium and cultured for 3 days at 37°C, 100% humidity, and microaerobic conditions (5% O₂, 12% CO₂, 83% N₂), and the resulting colonies were shake-cultured in 100 mL of Brucella broth (containing 10 mL of inactivated FBS and 0.4 mL of SR0147 *Helicobacter pylori* Selective Supplement (Dent)) at 37°C, 100% humidity, and microaerobic conditions (5% O₂, 12% CO₂, 83% N₂) for 3 days, and then the cells were collected by centrifugation. The cells were suspended in PBS and administered orally to 4-week-old female C57BL/6 mice at 2 × 10⁸ CFU three times every other day. DNA was extracted from the gastric mucosa of mice infected with *H. suis,* and the infection was confirmed by PCR. In addition, a solution obtained by suspending gastric mucosa of mice infected with *H. pylori* in a buffer solution was spread onto Nissui Helicobacter agar medium (Nissui Pharmaceutical Co., Ltd.) and cultured to confirm infection. Serum was collected from mice infected with each of *H. pylori* and *H. suis* 4 weeks after the last infection. As a control, serum was collected from healthy mice not infected with either virus (uninfected).

A suspension of *H. suis* whole cells (4 µg/mL) or *a H. suis* solubilized fraction (4 µg/mL) dissolved in 0.1 M carbonate-bicarbonate buffer (pH 9.4) was dropped at 100 µL/well onto a 96-well NUNC ImmunoPlate #439454 and left at 4°C overnight. The next day, the cell solution was discarded and washing was performed three times with 200 µL/well of PBS-T. A blocking solution (1% BSA, PBS-based, pH 7.4) prepared from Blocker BSA 10X in PBS (Thermo Fisher Scientific Inc.) was dropped at 200 µL/well and left at 37°C for 1 hour. The blocking solution was discarded, and washing was performed three times with 200 µL/well of PBS-T. Mouse serum diluted with the blocking solution was dropped at 50 µL/well and left at 37°C for 1 hour. The serum solution was discarded and washing was performed three times with 200 µL/well of PBS-T. Horseradish peroxidase labeled secondary antibody (Goat Anti-Mouse Ig, Human ads-HRP, SouthernBiotech, Inc.) diluted 100,000-fold with blocking solution was dropped at 50 µL/well and left at 37°C for 1 hour. The secondary antibody solution was discarded and washing was performed three times with 200 µL/well of PBS-T. SuperBlue TMB Microwell Peroxidase Substrate (1-Component) was dropped at 50 µL/well to generate a blue color, and then 1 N hydrochloric acid was dropped at 50 µL/well to change the color to yellow. The absorbance at 450 nm (reference wavelength: 620 nm to 630 nm) was measured using a plate reader.

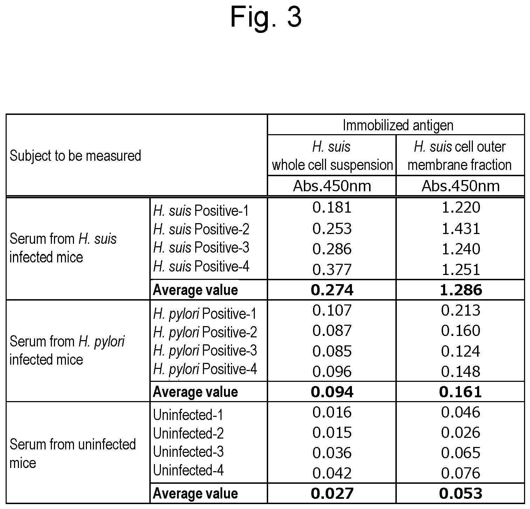

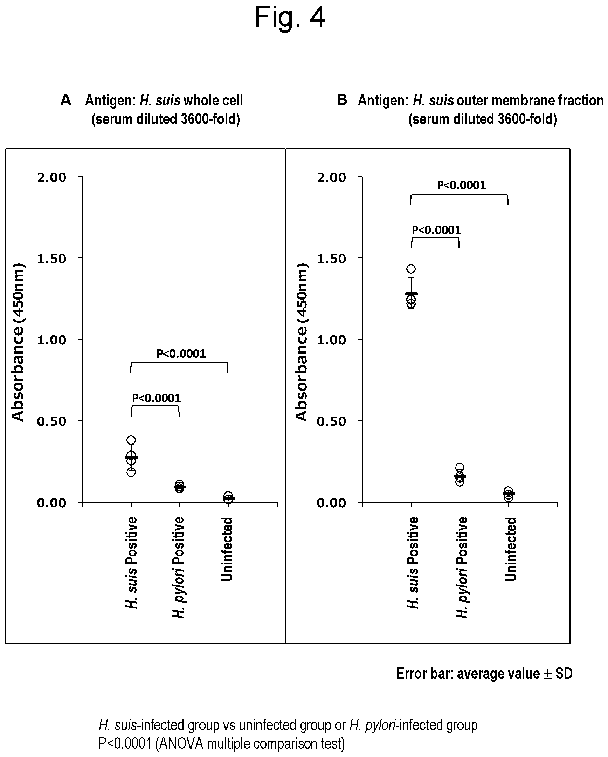

The results of ELISA using mouse serum (diluted 3600-fold) are shown in Figures 3 and 4. In the *H. suis*-positive subjects, absorbance was obtained for both antigens of the *H. suis* whole cell and the *H. suis* outer membrane fraction, but a higher absorbance was obtained for the *H. suis* outer membrane fraction. In addition, *H. pylori-*infected mice and uninfected mice not infected with *H. suis* showed low absorbance for both antigens. This showed for the first time that an antibody against the *H. suis* outer membrane fraction is produced in the blood of mice infected with *H. suis,* and that the use of the *H. suis* outer membrane fraction as an antigen allows the antibody to be captured with high sensitivity. This demonstrated that the use of the *H. suis* outer membrane fraction allows for distinction from *H. pylori* infection in mice and thus a highly sensitive diagnosis of *H. suis* infection.

### (Example 3) Measurement in human specimens

Measurement of anti-*H*. *pylori* antibody titers and anti-*H*. *suis* antibody titers in infected individuals (humans) using enzyme linked immunosorbent assay (ELISA)

### (1) Preparation of H. pylori antigens for ELISA (H. pylori cell outer membrane fraction)

*H. pylori* TN2GF4 strain (Non Patent Literature 2) was shake-cultured in Brucella broth containing 10% (v/v) fetal calf serum (FCS) at 37°C, 100% humidity, and microaerobic conditions (5% O₂, 10% CO₂, 85% N₂) for 48 hours, and then the culture was centrifuged (13,420 G × 10 min) to collect the cells. The cells were washed twice with phosphate buffered saline PBS, pH 7.4), suspended in distilled water and subjected to ultrasonic disruption treatment for 5 minutes under ice-cooled conditions (30 seconds of sonication and 30 seconds of rest were repeated five times using a Bioruptor II ultrasonic cell disrupter (setting High)). The disrupted solution was used as the *"H. pylori* whole cell suspension" and the sediment obtained by centrifugation (30,190 G × 10 minutes) was used as the *"H. pylori* cell outer membrane fraction", which was then suspended in distilled water. The protein was quantified using the Bio-Rad Protein Assay kit with BSA as the standard protein.

### (2) Preparation of H. suis antigen (H. suis cell outer membrane fraction) for ELISA Composition of H. suis medium

Brucella broth (BD BBL) 2.8 g
Agar powder (BD BBL) 1.5 g
Pyruvic acid (Sigma-Aldrich Co. LLC) 0.1 g, added for preparation of agar medium
Skirrow supplement (2 mL/vial, Oxoid Ltd.) 0.4 mL
Vitox supplement (10 mL/vial, Oxoid Ltd.) 2 mL
Amphotericin B (0.25 mg/mL) 2 mL
Concentrated hydrochloric acid 0.135 mL, for adjusting pH to 5
Inactivated fetal bovine serum (FBS) 20 mL
Distilled water, added to bring the total volume to 100 mL.

Nine mL of agar medium was added to a 75 cm² flask to prepare a slant medium, and *H. suis* SNTW101c strain (International Publication No. WO 2019/225639) stored at -80°C and isolated from a patient with goosebump gastritis was inoculated, 12 mL of liquid medium was added, and shaking culture was performed for one week at 37°C, 100% humidity, and microaerobic conditions (5% O₂, 12% CO₂, 83% N₂). Thereafter, the culture medium was centrifuged (13,420 G × 10 minutes) to collect the cells. The cells were washed twice with phosphate buffered saline (PBS, pH 7.4), suspended in distilled water, and subjected to ultrasonic disruption treatment for 5 minutes under ice-cooled conditions (30 seconds of sonication and 30 seconds of rest were repeated five times using a Bioruptor II ultrasonic cell disrupter (setting High)). The disrupted solution was used as *"H. suis* whole cell suspension", and the sediment obtained by centrifugation (30,190 G × 10 min) was used as "*H*. *suis* cell outer membrane fraction", which was then suspended in distilled water. Protein was quantified using the Bio-Rad Protein Assay kit with BSA as the standard protein.

An *H. pylori* cell outer membrane fraction and *H. suis* cell outer membrane fraction (4 µg/mL) dissolved in 0.05 M carbonate-bicarbonate buffer (pH 9.6) was dropped at 100 µL/well onto a 96-well NUNC ImmunoPlate #468667 and left at 4°C overnight. The next day, the cell solution was discarded and washing was performed three times with 250 µL/well ofPBS-T (PBS containing 0.05% (V/V) Tween 20). A blocking solution (1% (V/V) BSA, PBS-based, pH 7.0) was dropped at 200 µL/well and left at 37°C for 1 hour. The blocking solution was discarded, and washing was performed three times with 250 µL/well of PBS-T. Serum was each collected from *H. pylori-*infected individuals with symptoms of gastric disease (determined by existing testing methods (urea breath test, antibody test, antigen test, and the like)) and *H. suis*-infected individuals with symptoms of gastric disease (DNA was prepared from the subjects' gastric biopsies, and *H. suis* infection was determined by the real-time PCR method described in Patent Literature 2 or by culturing the gastric biopsies). A serum was collected from healthy subjects who were not infected with either bacterium (uninfected) as a control. Human serum diluted with specimen diluent (0.5% (v/v) BSA, PBS-based, pH 7.0) was dropped at 50 µL/well and left at 37°C for 1 hour. The serum solution was discarded and washing was performed three times with 250 µL/well of PBS-T. Horseradish peroxidase labeled secondary antibody (Goat anti-Human IgG (H+L), Jackson ImmunoResearch, Inc.) diluted 20,000-fold using secondary antibody diluent (1.0% (V/V) BSA, PBS-based, pH 7.0) was dropped at 50 µL/well and left at 37°C for 1 hour. The secondary antibody solution was discarded and washing was performed three times with 250 µL/well of PBS-T. TMB One Component HRP Microwell Substrate (Surmodics, Inc.) was dropped at 50 µL/well to generate a blue color, and then 0.17 M sulfuric acid was dropped at 50 µL/well to change the color to yellow. The absorbance was measured at 450 nm (reference wavelength: 620 nm to 630 nm) using a plate reader.

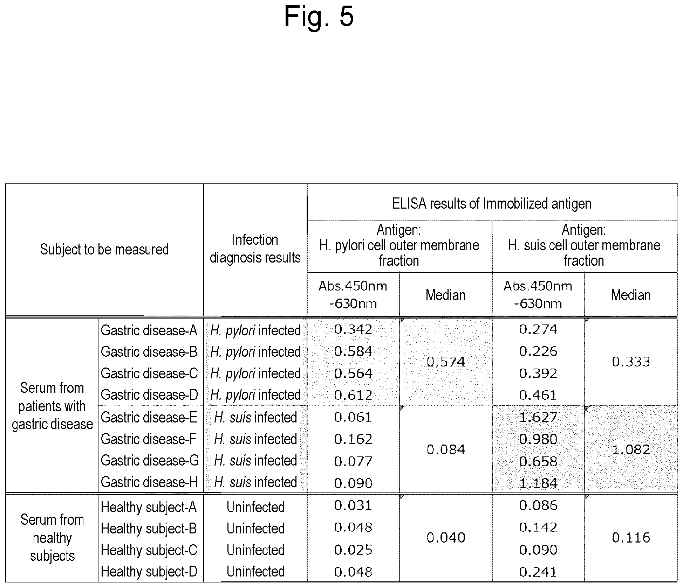

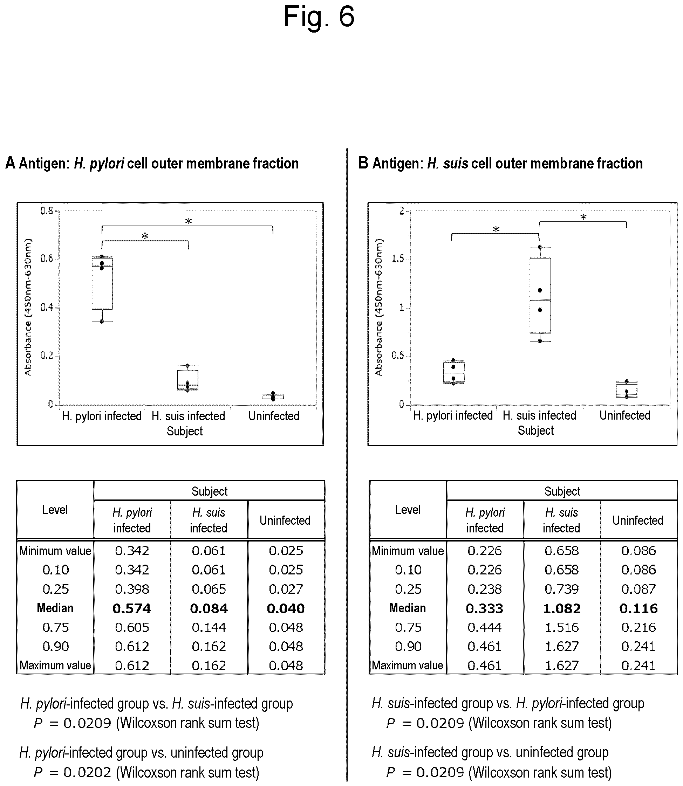

The results of ELISA using human serum (diluted 3600-fold) are shown in Figures 5 and 6. For the outer membrane fraction of *H. pylori* cell, the absorbance of the *H. pylori-*infected group (gastric disease, A, B, C, and D) was significantly higher than that of the *H. suis*-infected group (gastric disease, E, F, G, and H) or uninfected group (healthy subjects, A, B, C, and D) (*H. pylori-*infected group vs. *H. suis*-infected group: P = 0.0209, *H. pylori-*infected group vs. uninfected group: P = 0.0202). In addition, for the *H. suis* cell outer membrane fraction, the absorbance of the *H. suis*-infected group (gastric disease, E, F, G, and H) was significantly higher than that of the *H. pylori-*infected group (gastric disease, A, B, C, and D) or uninfected group (healthy subjects, A, B, C, and D) (*H. suis*-infected group vs. *H*. *pylori*-infected group or uninfected group: P = 0.0209). This has demonstrated that testing the same subject for both anti-H. *pylori* and anti-*H*. *suis* antibodies can determine not only whether or not a subject is infected with *H. pylori,* but also to diagnose *H. suis* infection in subjects who have previously been overlooked as *H. pylori* negative.

### (Example 4) Measurement in human specimen

Measurement of anti-*H*. *suis* antibody titers in infected individuals (humans) using enzyme linked immunosorbent assay (ELISA)
Composition of *H. suis* medium
Brucella broth (BD BBL) 2.8 g
Agar powder (BD BBL) 1.5 g
Pyruvic acid (Sigma-Aldrich Co. LLC) 0.1 g, added for preparation of agar medium
Skirrow Supplement (2 mL/vial, Oxoid Ltd.) 0.4 mL
Vitox Supplement (10 mL/vial, Oxoid Ltd.) 2 mL
Amphotericin B (0.25 mg/mL) 2 mL
Concentrated hydrochloric acid 0.135 mL, for adjusting pH to 5
Inactivated fetal bovine serum (FBS) 20 mL
Distilled water added to bring the total volume to 100 mL

Nine mL of agar medium was added to a 75 cm² flask to prepare a slant medium, and *H. suis* SNTW101c strain (Non Patent Literature 2) stored at -80°C and isolated from a patient with goosebump gastritis was inoculated, 12 mL of liquid medium was added, and shaking culture was performed at a temperature of 37°C, humidity of 100%, and under microaerobic conditions (5% O₂, 12% CO₂, 83% N₂) for one week. Thereafter, the culture medium was centrifuged (13,420 G × 10 minutes) to collect the cells. The cells were washed twice with phosphate buffered saline PBS, pH 7.4), suspended in distilled water, and subjected to ultrasonic disruption treatment for 5 minutes under ice-cooled conditions (30 seconds of sonication and 30 seconds of rest were repeated five times using a Bioruptor II ultrasonic cell disrupter (setting High)). The disrupted solution was used as *"H. suis* whole cell suspension", and the sediment obtained by centrifugation (30,190 G × 10 min) was used as "*H*. *suis* cell outer membrane fraction", which was then suspended in distilled water. Protein was quantified using the Bio-Rad Protein Assay kit with BSA as the standard protein.

The HsvA antigen peptides (11 types) show 14 amino acid residues that are a part of HsvA (an outer membrane protein that is specifically present only in *H. suis* and is made of approximately 3,000 amino acid residues) disclosed in International Publication WO2019/225639.
EKKAVQQMENSNPD (Peptide No. 11: SEQ ID NO: 1),
EKKAVEQMENSNPD (Peptide No. 11 (TKY): SEQ ID NO: 2),
EKDAVTSLKNSNSG (Peptide No. 11 (SH8): SEQ ID NO: 3),
EKDAVTSLENSNSG (Peptide No. 11 (SH10): SEQ ID NO: 4),
NQGTLEFLSNDVST (Peptide No. 19 (TKY): SEQ ID NO: 5),
TNGQEVS ASIDYNK (Peptide No. 16: SEQ ID NO: 6),
AKLSNFASNDALPD (Peptide No. 23: SEQ ID NO: 7),
PTTSSGASPDSSNP (Peptide No. 10: SEQ ID NO: 8),
NVDNILNMPSTTSG (Peptide No. 20: SEQ ID NO: 9),
TLTLEGTETFAQNS (Peptide No. 81: SEQ ID NO: 10), and
ADIQSSQTTFANSV (Peptide No. 61: SEQ ID NO: 11).

An *H. pylori* cell outer membrane fraction and HsvA antigen peptide (each 4 µg/mL) dissolved in 0.05 M carbonate-bicarbonate buffer (pH 9.6) were dropped at 100 µL/well onto a 96-well NUNC ImmunoPlate #468667 and left at 4°C overnight. The next day, the cell solution was discarded and washed three times with 250 µL/well of PBS-T (PBS containing 0.05% (V/V) Tween 20). A blocking solution (1% (V/V) BSA, PBS-based, pH 7.0) was dropped at 200 µL/well and left at 37°C for 1 hour. The blocking solution was discarded and washed three times with 250 µL/well of PBS-T. Serum was collected from *H. pylori-*infected individuals (determined by existing testing methods (urea breath test, antibody test, antigen test, and the like)) and *H. suis*-infected individuals (DNA was prepared from a gastric biopsy specimen of the subject, and *H. suis* infection was determined by the real-time PCR method described in Patent Literature 2 or by culturing the gastric biopsy specimen). As a control, serum was collected from healthy subjects not infected with either cell (uninfected). Human serum diluted with specimen diluent (0.5% (v/v) BSA, PBS-based, pH 7.0) was dropped at 50 µL/well and left at 37°C for 1 hour. The serum solution was discarded and washed three times with 250 µL/well of PBS-T. Horseradish peroxidase-labeled secondary antibody (Goat anti-Human IgG (H+L), Jackson ImmunoResearch, Inc.) diluted 20,000-fold using secondary antibody diluent (1.0% (V/V) BSA, PBS-based, pH 7.0) was dropped at 50 µL/well and left at 37°C for 1 hour. Horseradish peroxidase labeled secondary antibody (Goat anti-Human IgG (H+L), Jackson ImmunoResearch, Inc.) diluted 20,000-fold using secondary antibody diluent (1.0% (V/V) BSA, PBS-based, pH 7.0) was dropped at 50 µL/well and left at 37°C for 1 hour. TMB One Component HRP Microwell Substrate (Surmodics, Inc.) was dropped at 50 µL/well to generate a blue color, and then 0.17 M sulfuric acid was dropped at 50 µL/well to change the color to yellow. A plate reader measured the absorbance at 450 nm (reference wavelength: 620 nm to 630 nm).

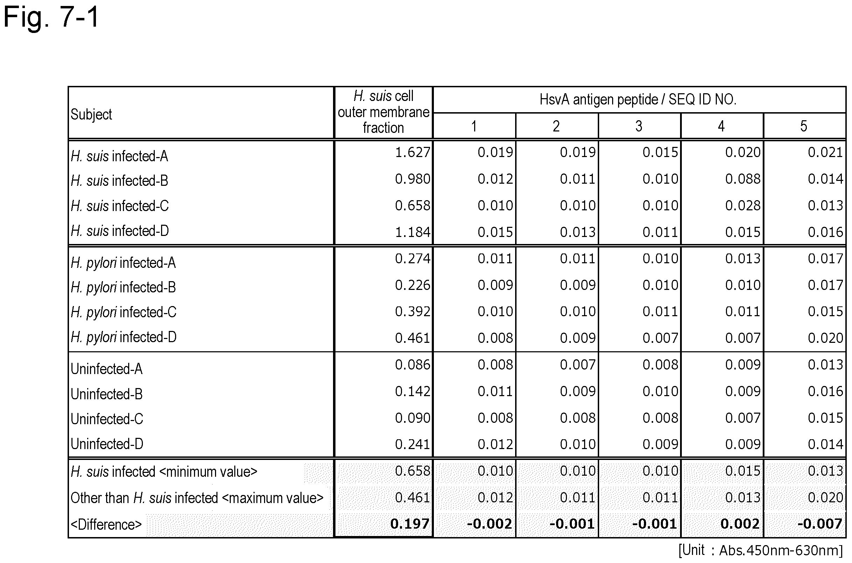

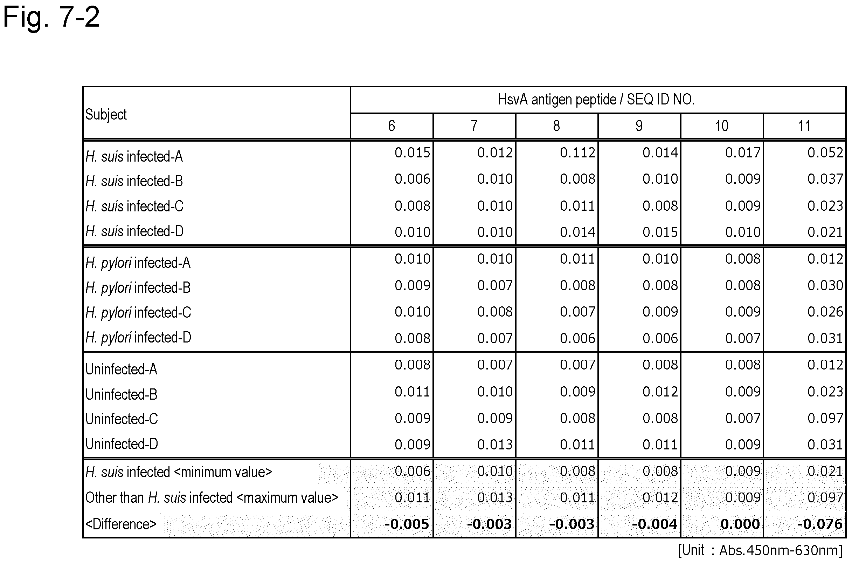

The results of ELISA using human serum (diluted 3600-fold) are shown in Figures 7-1 and 7-2. For the *H. suis* cell outer membrane fraction, even the specimen with the lowest absorbance for *H. suis*-infected individuals had a high absorbance of 0.658 (Abs. 450 nm to 630 nm), whereas the specimen with the highest absorbance for *H. pylori-*infected individuals and uninfected individuals not infected with *H. suis* only had a value of 0.461 (Abs. 450 nm to 630 nm), allowing to provide a clear distinction between *H. suis*-infected and uninfected individuals. In contrast, for the HsvA antigen peptide, no high absorbance was obtained for *H. suis*-infected individuals for any of the sequence peptides, failing to provide a clear distinction in the absorbances between *H. pylori-*infected individuals and uninfected individuals not infected with *H. suis.* This has demonstrated that the use of the *H. suis* cell outer membrane fraction allows for the diagnosis of *H. suis* with superior sensitivity and specificity to the detection system using the HsvA antigen peptide.

### Industrial Applicability

The method of the present invention allows highly sensitive diagnosis of *H. suis* infection with distinction from *H. pylori* infection.

All publications, patents, and patent applications cited herein are hereby incorporated by reference in their entirety.

### Sequence table free text

SEQ ID NO: 1 to 11: Synthesis

## Claims

1. A reagent for detecting an antibody that binds to *a H. suis* outer membrane fraction, or a protein contained in the outer membrane fraction, in a specimen derived from a subject, the reagent comprising at least the *H. suis* outer membrane fraction, or the protein contained in the outer membrane fraction and an anti-Ig antibody.

2. The reagent, according to claim 1, wherein the subject is a mammal.

3. The reagent, according to claim 1 or 2, wherein the specimen is derived from blood.

4. The reagent, according to any one of claims 1 to 3, is for detecting also an antibody that binds to *H. pylori* or an antigen of an *H. pylori* cell component, in the specimen derived from a subject, the reagent further comprising an *H. pylori* cell component or an antibody against the *H. pylori* cell component.

5. The reagent according to any one of claims 1 to 4, wherein the reagent is a reagent for ELISA or immunochromatography.

6. A method for detecting an antibody that binds to an anti-*H*. *suis* outer membrane fraction, or a protein contained in the outer membrane fraction, in a specimen derived from a subject, the method comprising the steps of:
(a) contacting a specimen derived from a subject with *a H. suis* outer membrane fraction, or a protein contained in the outer membrane fraction; and
(b) detecting an antibody bound to the *H. suis* outer membrane fraction, or the protein contained in the outer membrane fraction, in the specimen.

7. The method according to claim 6, wherein the subject is a mammal.

8. The method according to claim 6 or 7, wherein the specimen is derived from blood.

9. The method according to any one of claims 6 to 8, for detecting also an antibody that binds to *H. pylori* in the specimen derived from a subject, the method further comprising the steps of
(a) contacting a specimen derived from a subject with an *H. pylori* cell component; and
(b) detecting an antibody bound to the *H. pylori* cell component in the specimen.

10. The method according to any one of claims 6 to 8, for detecting also an antigen of an *H. pylori* cell component in the specimen derived from a subject, the method further comprising the steps of
(a) contacting a specimen from a subject with an antibody against an *H. pylori* cell component; and
(b) detecting an antigen of an *H. pylori* cell component, bound to the antibody against the *H. pylori* cell component in the specimen.

11. A method for detecting infection with *H. suis,* wherein a subject is determined to be infected with *H. suis,* in which an antibody is detected by a measurement using the reagent according to any one of claims 1 to 5 and/or the method according to any one of claims 6 to 10.
